# EUROPEAN PATENT APPLICATION

(11) **EP 4 679 054 A1**
(43) Date of publication of application: **14.01.2026**
(21) Application number: 24903897.7
(22) Date of filing: 04.09.2024
(51) Int. Cl.: G01N 1/22, G01N 33/00, H01M 10/42, B65G 47/80, B65G 21/20, B65G 47/90

(54) **GAS COLLECTION DEVICE**

(30) Priority: 15.12.2023 KR 20230183126
(71) Applicant: LG ENERGY SOLUTION, LTD., Seoul 07335 (KR)
(72) Inventor: MOON, Minkook, Daejeon 34122 (KR); KIM, Kyung Min, Daejeon 34122 (KR); PARK, Kwangyeon, Daejeon 34122 (KR); CHOI, Nak Hee, Daejeon 34122 (KR)
(74) Representative: Goddar, Heinz J.
(86) International application number: PCT/KR2024/013306
(87) International publication number: WO 2025/127326

(57) **Abstract**

Disclosed is a gas collection device capable of precisely and stably transferring collected gases to be analyzed to a gas analysis module by automating the loading, unloading, punching, and the like of a plurality of batteries.

## Description

### Technical Field

The present application claims the benefit of priority based on Korean Patent Application No. 10-2023-0183126 filed on December 15, 2023, the entire disclosure of which is incorporated as a part of this specification.

The present application relates to an apparatus for collecting gas, and more particularly, to an apparatus for collecting gas capable of accurately and stably delivering collected analysis target gas to a gas analysis module by automating loading, unloading, and punching of a battery for multiple secondary batteries.

### Background

Secondary batteries are batteries that can be used repeatedly through a discharging process that converts chemical energy into electrical energy and a charging process that converts electrical energy into chemical energy, and commonly known types include nickelcadmium (Ni-Cd) batteries, nickel-metal hydride (Ni-MH) batteries, lithium-metal batteries, lithium-ion (Li-ion) batteries, and lithium-ion polymer batteries, etc. Among these secondary batteries, lithium secondary batteries have been commercialized and are widely used due to their high energy density and voltage, long cycle life, and low self-discharge rate.

Various types of gases may be generated inside a lithium secondary battery depending on the charge and discharge reaction, such as hydrogen, oxygen, nitrogen, carbon monoxide, carbon dioxide, hydrocarbons such as CₙH₂ₙ₋₂ (n=2-5), CₙH₂ₙ (n=2-5), and CₙH₂ₙ₊₂ (n=1-5), and other organic gas species.

In addition, lithium secondary batteries generate a large amount of gas in the process of electrolyte decomposition and degradation due to repeated charging and discharging, and this aspect varies depending on the design and usage of the battery. Therefore, it is essential to analyze the gas generated inside the battery to infer a degradation mechanism of the battery during the battery development process.

Therefore, it is very important to collect and accurately analyze the gas generated inside the secondary battery. Information on the composition and content of various gases generated during charging and discharging of lithium secondary batteries is useful for developing battery materials, optimizing the battery manufacturing process, and identifying the cause of battery failure. To this end, the development of technology to collect gas generated inside the secondary battery is important.

The following process may be performed as one of methods for analyzing the gas generated inside the secondary battery.

In order to collect an analysis target gas generated inside the secondary battery, a perforation hole is formed in the case of the secondary battery, and the analysis target gas is extracted through the perforation hole.

The extracted analysis target gas is diffused into a sealed gas diffusion space.

The analysis target gas diffused in the gas diffusion space is sampled in a sampling vessel.

The analysis target gas sampled in the sampling vessel is injected into a gas analysis apparatus such as gas chromatography-mass spectrometry (GC-MS) to perform gas analysis.

In order to obtain reliable analysis results, it is necessary to collect data by performing the above processes on multiple batteries. However, if the above processes are performed manually, reproducibility decreases, and issues such as the risk of gas leakage and contamination of the apparatus due to repeated operations may occur. Therefore, an automated gas collecting technology for secondary battery analysis is required to solve the above problems.

### Disclosure of the Invention

### Technical Goals

The present disclosure relates to a gas collecting apparatus, and is directed to providing a gas collecting apparatus capable of accurately and stably collecting analysis target gas and delivering it to a gas analysis module by automating loading, unloading, and punching of a battery for multiple secondary batteries.

Technical problems to be achieved by the present disclosure are not limited to the technical problems mentioned above, and other technical problems not mentioned may be clearly understood by those skilled in the art to which the present disclosure pertains from the description below.

### Technical Solutions

A gas collecting apparatus may include:
a loading unit configured to sequentially supply a plurality of analysis target batteries, one by one, to a first position on a first moving unit,
wherein the first moving unit is configured to move an analysis target battery of the plurality of batteries located at the first position to a second position on the first moving unit;
a gripper unit configured to position the analysis target battery located at the second position in a gas extraction unit or move the analysis target battery positioned in the gas extraction unit to the second position,
wherein the gas extraction unit is configured to extract gas from the battery housed therein;
a second moving unit configured to receive the analysis target battery located at the second position from the first moving unit and move the battery to a third position, wherein the third position is located on the second moving unit; and
an unloading unit configured to receive the analysis target battery located at the third position from the second moving unit.

According to an embodiment, the first moving unit may include a first rotating part configured to rotate around a first center as a central axis, and a first driving part configured to rotate the first rotating part.

According to an embodiment, the first rotating part may have a disk shape, and the first moving unit may further include a first guide part configured to restrict the analysis target battery from deviating from a circumferential direction of the first rotating part.

According to an embodiment, the loading unit may include a loading conveyor belt configured to sequentially transport the plurality of analysis target batteries to an upper surface of the first rotating part, and a loading driver configured to drive the loading conveyor belt, and an upper surface of the loading conveyor belt may be higher than an upper end part of the first guide part.

According to an embodiment, the loading unit may further include a pair of loading guide bars configured to guide the plurality of analysis target batteries so as to be aligned along a longitudinal direction of the loading conveyor belt and restrict the plurality of batteries from leaving the loading conveyor belt, and the pair of loading guide bars may be parallel to each other, spaced apart from each other by a distance greater than or equal to a diameter of each battery of the plurality of analysis target batteries, and extend in the longitudinal direction of the loading conveyor belt.

According to an embodiment, the loading conveyor belt may be configured to transport the plurality of analysis target batteries in a first direction, and the second moving unit may include a second rotating part configured to rotate around a second center as a central axis that is spaced apart from the first center by a predetermined distance in a second direction, the second direction being perpendicular to the first direction and parallel to the upper surface of the first rotating part, and a second driving part configured to rotate the second rotating part.

According to an embodiment, the second rotating part may have a disk shape, and the predetermined distance may be smaller than a sum of a radius of the first rotating part and a radius of the second rotating part.

According to an embodiment, the predetermined distance may be a value obtained by subtracting a diameter of the analysis target battery from the sum of the radius of the first rotating part and the radius of the second rotating part, the second moving unit may further include a second guide part configured to restrict the analysis target battery from deviating from a circumferential direction of the second rotating part, and the first rotating part and the second rotating part overlap at a fourth position, and the fourth position may be a position where the first guide part and the second guide part are open to each other.

According to an embodiment, the unloading unit may include an unloading conveyor belt configured to sequentially receive the plurality of analysis target batteries from the second rotating part, and an unloading driver configured to provide driving force to the unloading conveyor belt, an upper surface of the unloading conveyor belt may be lower than an upper surface of the second rotating part, and the second guide part may be open in an area of the second rotating part that is in contact with one end part of the unloading conveyor belt.

According to an embodiment, the first position may be a position where the analysis target battery loaded by the loading conveyor belt onto the first rotating part is first placed, the first rotating part may be configured to rotate in a direction such that the analysis target battery located at the first position moves away from the fourth position, the second rotating part may be configured to rotate in an opposite direction to the first rotating part, and at the fourth position, the analysis target battery may be configured to move from the first rotating part to the second rotating part by the rotation of the second rotating part.

According to an embodiment, the second position may be a position before the analysis target battery located at the first position is rotated by the first rotating part and arrives at the fourth position, and the third position may be a position of an edge of the second rotating part where the second rotating part is in contact with the one end part of the unloading conveyor belt.

According to an embodiment, the gas extraction unit may include a lower jig into which a lower end part of the analysis target battery is configured to be inserted; an upper jig configured to cover an upper end part of the analysis target battery and couple with an upper end part of the lower jig; a punching part mounted on the upper jig and configured to form a perforation hole on an upper surface of the analysis target battery; a jig moving part configured to move the upper jig in a vertical direction; and a clump configured to maintain the upper jig and the lower jig in a coupled state.

According to an embodiment, each of the upper jig and the lower jig may have a cylindrical shape extending in the vertical direction, a first protrusion part protruding in a radial direction along an outer circumferential surface may be formed at a lower end part of the upper jig, a second protrusion part protruding in a radial direction along an outer circumferential surface may be formed at the upper end part of the lower jig, and the clump may be configured to hold the first protrusion part and the second protrusion part so as to be in close contact with each other in a state that a bottom surface of the first protrusion part and an upper surface of the second protrusion part are in close contact with each other.

According to an embodiment, the clumps may be provided in a pair, and a pair of the clumps may hold opposite ends of each other.

According to an embodiment, the gas collecting apparatus may further include a cleaning unit configured to clean an inner side of the upper jig and a punching needle of the punching part.

According to an embodiment, the cleaning unit may include a nozzle part configured to spray air; a friction block configured to clean through friction; a first frame having one end part coupled to the nozzle part and the other end part coupled to the friction block; a first rail configured to guide the first frame to move in a longitudinal direction of the first frame; a second frame extending in a direction different from the longitudinal direction of the first frame; and a second rail configured to guide the second frame to move in a longitudinal direction of the second frame, and the first rail may be fixed to the second frame, and the first frame may be configured to be moved toward or away from the upper jig by movement of the second frame.

According to an embodiment, the gas collecting apparatus may further include a gas diffusion unit having a gas diffusion space configured to receive the analysis target gas from the gas extraction unit and diffuse the analysis target gas.

According to an embodiment, the gas diffusion unit may include a base plate part having a plane shape perpendicular to a vertical direction; a cylindrical side wall part having a lower end part fixed to the base plate part, the cylindrical side wall part configured to be stretched in the vertical direction; a vertical moving part configured to move in the vertical direction, wherein an upper end part of the cylindrical side wall part is fixedly coupled to the vertical moving part. in the vertical direction; a guide support part configured to guide a movement of the vertical moving part; and a vertical driving part configured to move the vertical moving part in the vertical direction, and the gas diffusion space may be defined by the base plate part, the cylindrical side wall part and the vertical moving part.

According to an embodiment, a gas port may be formed in the base plate part, and the analysis target gas may be injected into or discharged from the gas diffusion space through the gas port.

According to an embodiment, the cylindrical side wall part may have a bellows structure.

According to an embodiment, the vertical moving part may include a body member with a cylindrical shape extending in the vertical direction, and an upper plate member formed as a plane perpendicular to the vertical direction and having a bottom surface to which an upper end part of the body member is fixedly coupled, the upper end part of the cylindrical side wall part may be fixedly coupled to the bottom surface of the upper plate member, the body member may be located inside the cylindrical side wall part, and the gas diffusion space may be formed as a space surrounded by a lower end part of the body member, an upper surface of the base plate part, and an inner circumferential surface of the cylindrical side wall part.

According to an embodiment, when the vertical moving part is lowered to a lowest point, the lower end part of the body member may contact the upper surface of the base plate part, and all inner circumferential surfaces of the cylindrical side wall part may face an outer circumferential surface of the body member.

According to an embodiment, the upper plate member may be formed in a disk shape, the guide support part may be formed in a cylindrical shape extending in the vertical direction,
an inner diameter of the guide support part may be the same as a diameter of the upper plate member, and the upper plate member may be guided by sliding on an inner circumferential surface of the guide support part.

According to an embodiment, a lower end part of the guide support part may be fixed to the upper surface of the base plate part, a guide hole extending in the vertical direction may be formed on a side surface of the guide support part, the vertical driving part may include a power transmission member in which one end part is inserted into the guide support part through the guide hole, a vertical moving shaft with the vertical direction as a longitudinal direction and coupled to the other end of the power transmission member located outside the guide support part, and a driving actuator that is supported on the base plate part and configured to move the vertical moving shaft in the vertical direction, and the power transmission member may be coupled to the upper surface of the upper plate member.

### Advantageous Effects

A gas collecting apparatus of the present disclosure may be capable of accurately and stably delivering collected analysis target gas to a gas analysis module by automating loading, unloading, and punching of a battery for multiple secondary batteries.

In addition, the gas collecting apparatus of the present disclosure may, when sampling the analysis target gas diffused in the gas diffusion space of the gas diffusion unit, sample the analysis target gas at an optimized concentration for analysis by the gas analysis unit by varying the volume of the gas diffusion space.

### Brief Description of Drawings

FIG. 1 is a schematic diagram illustrating a gas collecting apparatus according to an embodiment.
FIGS. 2 to 8 are schematic diagrams illustrating a movement order of an analysis target battery.
FIG. 9 is a perspective view illustrating a gas extraction unit according to an embodiment.
FIGS. 10 to 12 are plan views illustrating a cleaning unit according to an embodiment.
FIG. 13 is a side view illustrating a gas diffusion unit according to an embodiment.
FIGS. 14 and 15 are cross-sectional views illustrating a gas diffusion unit according to an embodiment.

### Best Mode for Carrying Out the Invention

Hereinafter, embodiments according to the present disclosure will be described in detail with reference to the accompanying drawings. In this process, the size or shape of the components shown in the drawings may be exaggerated for clarity and convenience of explanation. In addition, terms specifically defined in consideration of the configuration and operation of the present disclosure may vary according to the intentions or customs of users and operators. Definitions of these terms should be made based on the content throughout this specification.

In the description of the present disclosure, it should be noted that the orientation or positional relationship indicated by the terms such as "center", "upper", "lower", "left", "right", "vertical", "horizontal", "inner side", "outer side", "one surface", and "other surface" is based on the orientation or positional relationship shown in the drawing or the orientation or positional relationship normally arranged when using the product of the present disclosure, and it is intended only for explanation and brief description of the present disclosure, and is not to be construed as limiting the present disclosure as it does not suggest or imply that the device or element shown must necessarily be configured or operated in a specific orientation with a specific orientation.

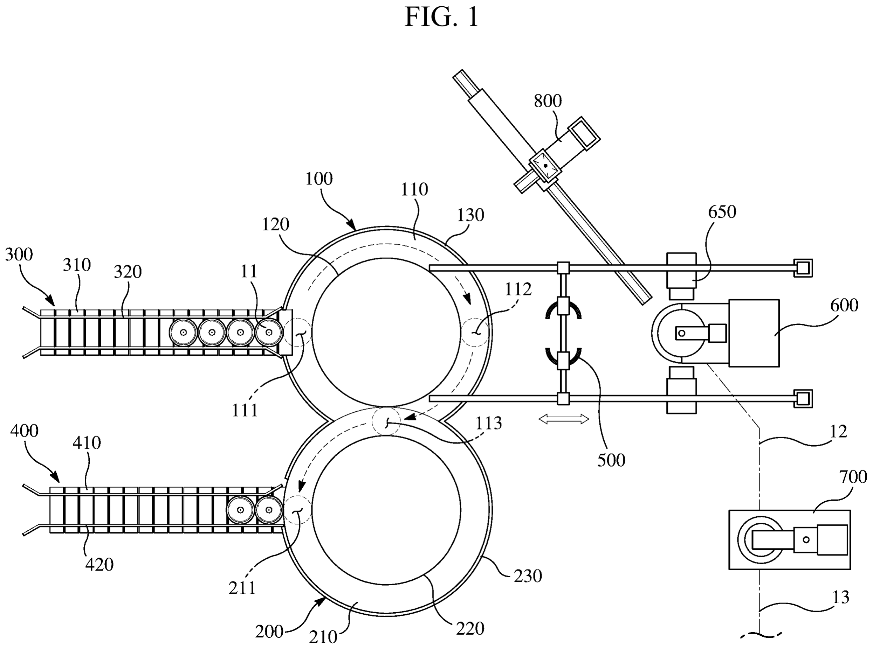

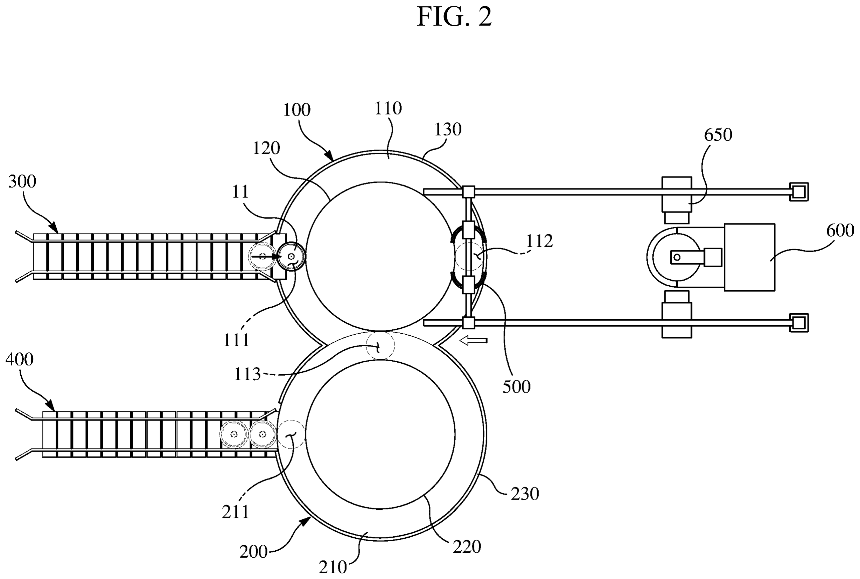

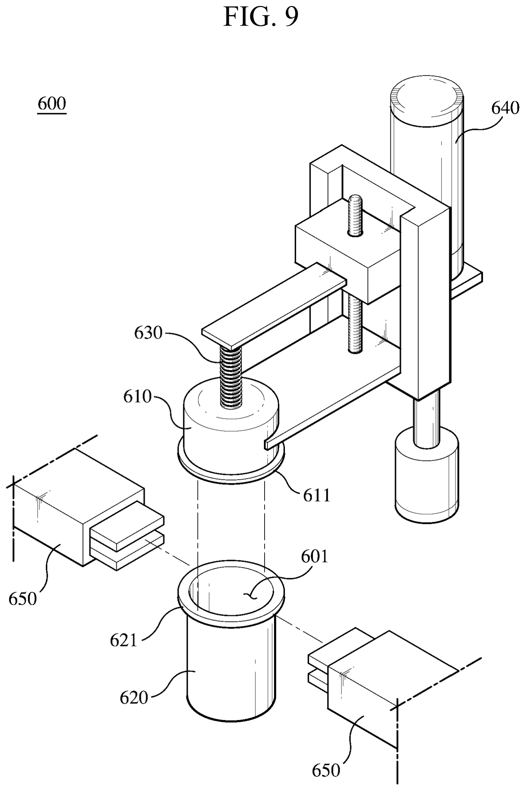

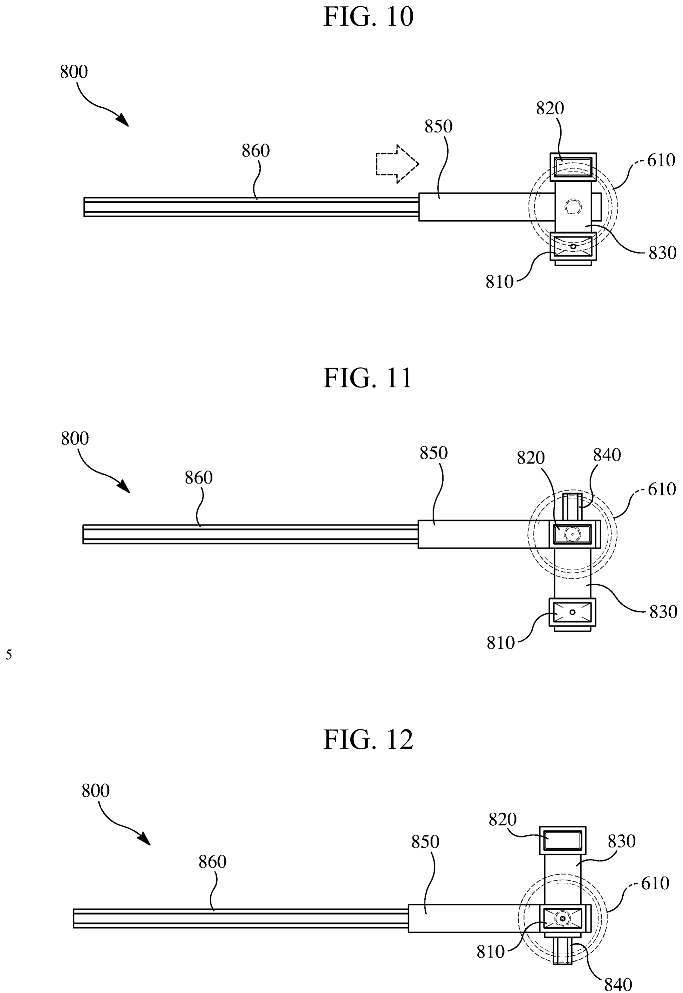

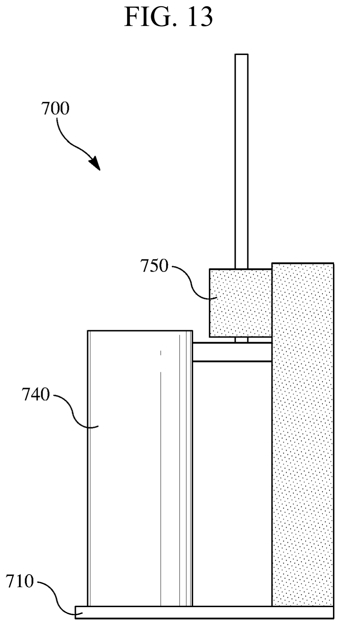

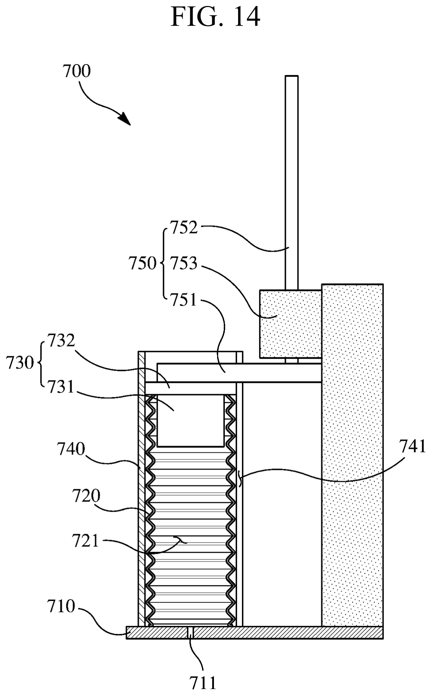

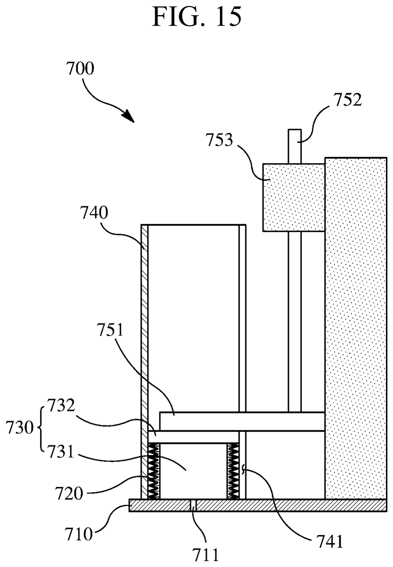

FIG. 1 is a schematic diagram illustrating a gas collecting apparatus according to an embodiment. FIGS. 2 to 8 are schematic diagrams illustrating a movement order of an analysis target battery 11 according to an embodiment. FIG. 9 is a perspective view illustrating a gas extraction unit 600 according to an embodiment. FIGS. 10 to 12 are plan views illustrating a cleaning unit 800 according to an embodiment. FIG. 13 is a side view illustrating a gas diffusion unit 700 according to an embodiment. FIGS. 14 and 15 are cross-sectional views illustrating the gas diffusion unit 700 according to an embodiment.

Hereinafter, the gas collecting apparatus of the present disclosure will be described in detail with reference to FIGS. 1 to 15.

The gas collecting apparatus may sequentially collect gas from a plurality of secondary batteries in an automated system. Specifically, the gas collecting apparatus of the present disclosure may automatically and sequentially perform processes such as punching, gas extraction, gas diffusion, gas sampling, and battery recovery for a plurality of secondary batteries without manual work.

The gas collecting apparatus may extract gas generated inside a secondary battery and deliver it to a device such as a gas chromatography-mass spectroscopy (GC-MS), a gas chromatography-pulsed discharge detector (GC-PDD), a gas chromatography-thermal conductivity detector (GC-TCD), a gas chromatography-flame ionization detector (GC-FID), etc.

As an embodiment, in the gas collecting apparatus, the analysis target battery 11 may be a secondary battery having a rigid cylindrical case.

In another embodiment, in the gas collecting apparatus, the analysis target battery 11 may be a secondary battery of various shapes housed in a chemically resistant jig with an open upper end part. The chemically resistant jig in which the secondary battery is housed may be provided with a cylindrical outer shape. The chemically resistant jig may be provided in plurality, and the plurality of chemically resistant jigs may be formed with different internal structures. The internal structures of the plurality of chemically resistant jigs may be formed to correspond to various types of secondary battery structures. When the plurality of secondary batteries are housed in the plurality of chemically resistant jigs, the upper end parts of each of the plurality of secondary batteries may be housed so as to be exposed.

In the following description, the vertical direction may be the direction of gravity. For example, the upper part may refer to a position having higher potential energy than the lower part.

As illustrated in FIG 1, the gas collecting apparatus of the present disclosure may include:
a gas extraction unit 600 configured to extract analysis target gas from the inside of the analysis target battery 11;
a loading unit 300 configured to sequentially supply a plurality of analysis target batteries 11 one by one to a first position 111;
a first moving unit 100 configured to move the analysis target battery 11 located at the first position 111 to a second position 112;
a gripper unit 500 configured to house the analysis target battery 11 located at the second position 112 in the gas extraction unit 600 or move the analysis target battery 11 housed in the gas extraction unit 600 to the second position 112;
a second moving unit 200 configured to receive the analysis target battery located at the second position 112 from the first moving unit 100 and move it to a third position 211; and
an unloading unit 400 configured to recover the analysis target battery 11 located at the third position 211.

In FIG. 1, a straight line indicated by a dashed dot line is a gas flow path, which may be a first gas flow path 12 and a second gas flow path 13. The analysis target gas generated in the gas extraction unit 600 may be delivered to a gas sampling unit or a gas analysis unit. Specifically, the analysis target gas initially extracted from the analysis target battery 11 in the gas extraction unit 600 may be sent to a gas diffusion unit 700 described below through the first gas flow path 12. In the gas diffusion unit 700, the analysis target gas may be adjusted to a concentration and pressure optimized for analysis and delivered to the gas sampling unit or the gas analysis unit through the second gas flow path 13.

The first gas flow path 12 and the second gas flow path 13 may be gas flow paths including a pipe, a hose, etc.

The gas sampling unit (not shown) may include a gas sampling vessel having a gas sampling space formed as a fixed volume inside, and a shut-off valve for opening and closing the gas sampling vessel.

The gas analysis unit (not shown) may be a gas chromatography-mass spectroscopy (GC-MS), a gas chromatography-pulsed discharge detector (GC-PDD), a gas chromatography-thermal conductivity detector (GC-TCD), a gas chromatography-flame ionization detector (GC-FID), etc.

The first moving unit 100 may include a first rotating part 110 rotating around a first center as a central axis, and a first driving part 120 for providing the driving force to rotate the first rotating part 110, and the loading unit 300 may supply the analysis target battery to the upper surface of the first rotating part 110. The first rotating part 110 may have a disk shape, and the first moving unit 100 may further include a first guide part 130 configured to prevent the analysis target battery 11 from leaving along a circumferential direction of the first rotating part 110.

More specifically, the first rotating part 110 is a plane perpendicular to the vertical direction and may be provided in a disk shape centered around the first center. The analysis target battery 11 is placed on the edge of the first rotating part 110, and the analysis target battery 11 may move in the circumferential direction of the first rotating part 110 according to the rotation of the first rotating part 110. On the edge of the first rotating part 110, a plurality of battery accommodating grooves having a shape corresponding to the lower end part of the analysis target battery may be formed along the circumferential direction.

The first driving part 120 may be a rotor. The first driving part 120 may have the vertical direction as the rotation axis. The first rotating part 110 may be rotated at the position of the first center.

The first guide part 130 may be provided in a ring shape. The first guide part 130 is formed along the edge of the first rotating part 110 and may not be formed in an area overlapping with the second rotating part 210 described later. The upper end part of the first guide part 130 may be formed higher than the upper surface of the first rotating part 110.

The loading unit 300 may include a loading conveyor belt 310 configured to sequentially transport the analysis target batteries 11 to the upper surface of the first rotating part 110, and a loading driver configured to provide the driving force to the loading conveyor belt 310. The height of the upper surface of the loading conveyor belt 310 may be higher than the upper end part of the first guide part 130.

In other words, the analysis target batteries 11 may be sequentially placed from the loading conveyor belt 310 to the first rotating part 110. The longitudinal direction of the loading conveyor belt 310 may be the radial direction of the first rotating part 110. One end part of the loading conveyor belt 310 may face the first center.

The loading unit 300 may further include a pair of loading guide bars 320 configured to guide the plurality of analysis target batteries 11 to be aligned in the longitudinal direction of the loading conveyor belt and prevent them from leaving the loading conveyor belt 310.

The pair of loading guide bars 320 may be parallel to each other, be spaced apart from each other by the distance greater than or equal to the diameter of the analysis target batteries 11, and extend in the longitudinal direction of the loading conveyor belt 310.

When the direction in which the loading conveyor belt 310 transports the analysis target batteries 11 is referred to as a first direction, and the direction perpendicular to the first direction and parallel to the upper surface of the first rotating part 110 is referred to as a second direction, the second moving unit 200 may include a second rotating part 210 rotating around a second center as a central axis that is spaced apart from the first center by a predetermined distance in the second direction, and a second driving part 220 configured to provide the driving force for rotating the second rotating part 210.

The second rotating part 210 may be provided in a disk shape. The second moving unit 200 may further include a second guide part 230 configured to prevent the analysis target batteries 11 from leaving along the circumferential direction of the second rotating part 210.

More specifically, the second rotating part 210 is a plane perpendicular to the vertical direction and may be provided in a disk shape centered around the second center. The analysis target battery 11 is placed on the edge of the second rotating part 210, and the analysis target battery 11 may move in the circumferential direction of the second rotating part 210 according to the rotation of the second rotating part 210. On the edge of the second rotating part 210, a plurality of battery accommodating grooves having a shape corresponding to the lower end part of the analysis target battery may be formed along the circumferential direction.

The second driving part 220 may be a rotor. The second driving part 220 may have the vertical direction as the rotation axis. The second rotating part 210 may be rotated at the position of the second center. The second driving part 220 may rotate in the opposite direction to the first driving part 120.

The second guide part 230 may be provided in a ring shape. The second guide part 230 is formed along the edge of the second rotating part 210 and may not be formed in an area overlapping the first rotating part 110 and an area facing the unloading conveyor belt. The upper end part of the second guide part 230 may be formed higher than the upper surface of the second rotating part 210.

The predetermined distance between the first center and the second center may be smaller than the sum of the radius of the first rotating part 110 and the radius of the second rotating part 210. More specifically, the predetermined distance may be a value obtained by subtracting the diameter of the analysis target batteries 11 from the sum of the radius of the first rotating part 110 and the radius of the second rotating part 210. In the area where the first rotating part 110 and the second rotating part 210 overlap, the first guide part 130 and the second guide part 230 may be open.

The unloading unit 400 may include an unloading conveyor belt 410 configured to sequentially recover the analysis target batteries 11 placed on the upper surface of the second rotating part 210, and an unloading driver configured to provide the driving force to the unloading conveyor belt 410, and the height of the upper surface of the unloading conveyor belt 410 may be lower than the height of the upper surface of the second rotating part 210.

On an arc of the second rotating part 210 that contacts one end part of the unloading conveyor belt 410, the second guide part 230 may be open.

The unloading unit 400 may further include a pair of unloading guide bars 420 configured to guide the plurality of analysis target batteries 11 to be aligned in the longitudinal direction of the unloading conveyor belt 410 and prevent them from leaving the unloading conveyor belt 410.

The pair of unloading guide bars 420 may be parallel to each other, be spaced apart from each other by the distance greater than or equal to the diameter of the analysis target batteries 11, and extend in the longitudinal direction of the unloading conveyor belt 410.

The first position 111 may be a position where the analysis target batteries 11 loaded by the loading conveyor belt 310 on the first rotating part 110 are first placed. When the area where the first rotating part 110 and the second rotating part 210 overlap is referred to as a fourth position 113, the first rotating part 110 may rotate in a direction in which the analysis target battery 11 located at the first position 111 moves away from the fourth position 113. For example, the angle formed by the first position 111 and the fourth position 113 with the first center as the vertex may be a right angle. Here, when designating the first position 111, the fourth position 113, and the second position 112, the third position 211 as point positions, it means the center of the corresponding position. The meaning that the first rotating part 110 rotates in a direction in which the analysis target battery 11 located at the first position 111 moves away from the fourth position 113 means that the angle formed by the fourth position 113 and the analysis target battery 11 with the first center as the vertex gradually increases.

The second rotating part 210 may rotate in the opposite direction to the first rotating part 110.

In the area where the first rotating part 110 and the second rotating part 210 overlap, the analysis target battery 11 may be moved to the second rotating part 210 and moved by the rotation of the second rotating part 210. In other words, at the fourth position 113, the analysis target battery 11 may be moved by the second rotating part 210. More specifically, when the analysis target battery 11 reaches the fourth position 113, the analysis target battery 11 may be moved to the third position 211 by the second rotating part 210.

The second position 112 may be the position before the analysis target battery 11 located at the first position 111 is rotated by the first rotating part 110 and arrives at the fourth position 113, and the third position 211 may be the position of the edge of the second rotating part 210 that contacts one end part of the unloading conveyor belt 410.

For example, the second position 112 may be a position rotated 180° in the rotational direction of the first rotating part 110 from the first position 111.

For example, the third position 211 may be a position rotated 90° in the rotational direction of the second rotating part 210 from the fourth position 113.

Hereinafter, with reference to FIGS. 2 to 9, the movement process of the analysis target battery 11 will be specifically described.

As illustrated in FIG. 2, the analysis target battery 11 may be loaded from the loading unit 300 to the first position 111 of the first moving unit 100.

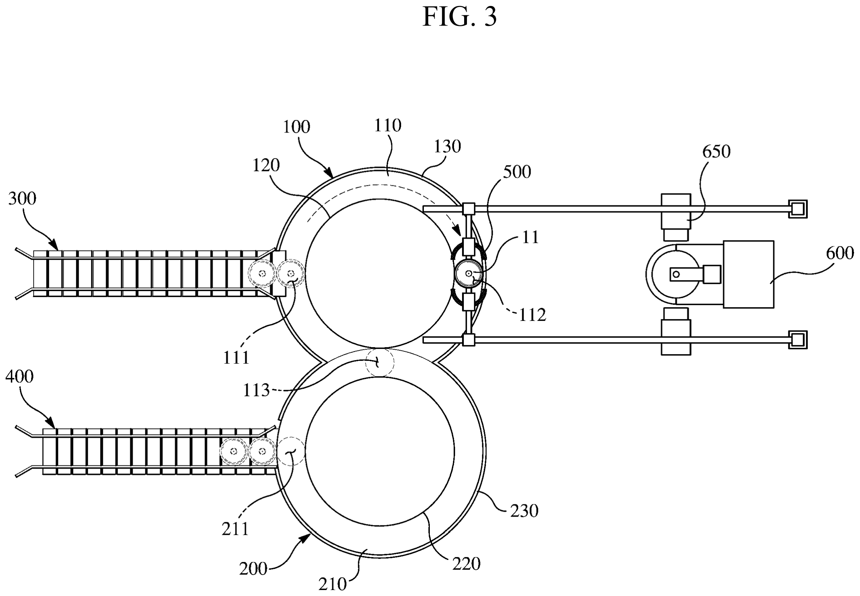

As illustrated in FIG. 3, the analysis target battery 11 may be moved from the first position 111 to the second position 112 by the rotation of the first rotating part 110.

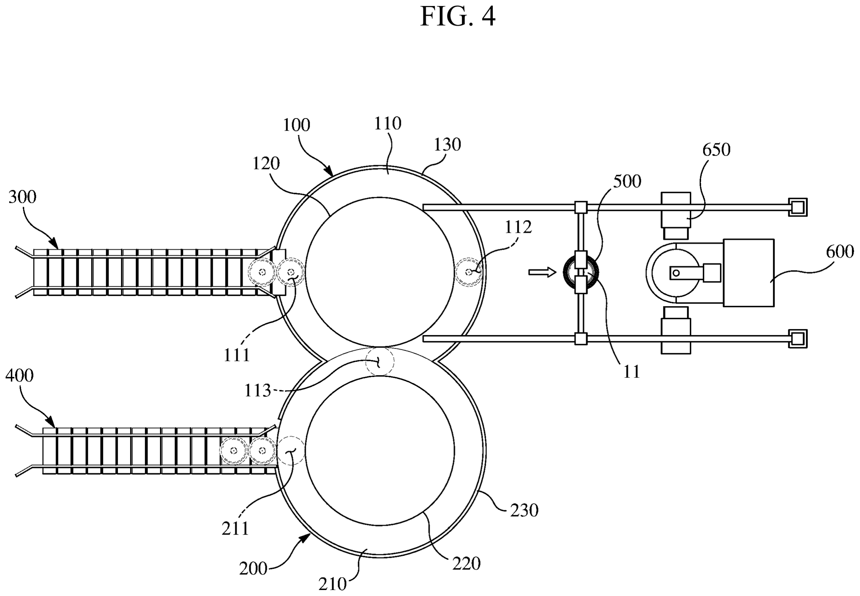

As illustrated in FIG. 4, the analysis target battery 11 may be moved from the second position 112 to the gas extraction unit 600 by the gripper unit 500. The gripper unit 500 may include a gripping part configured to grip the analysis target battery 11 and a transporting part configured to transport the gripping part. The transporting part may be capable of moving in at least two axes, including the vertical direction.

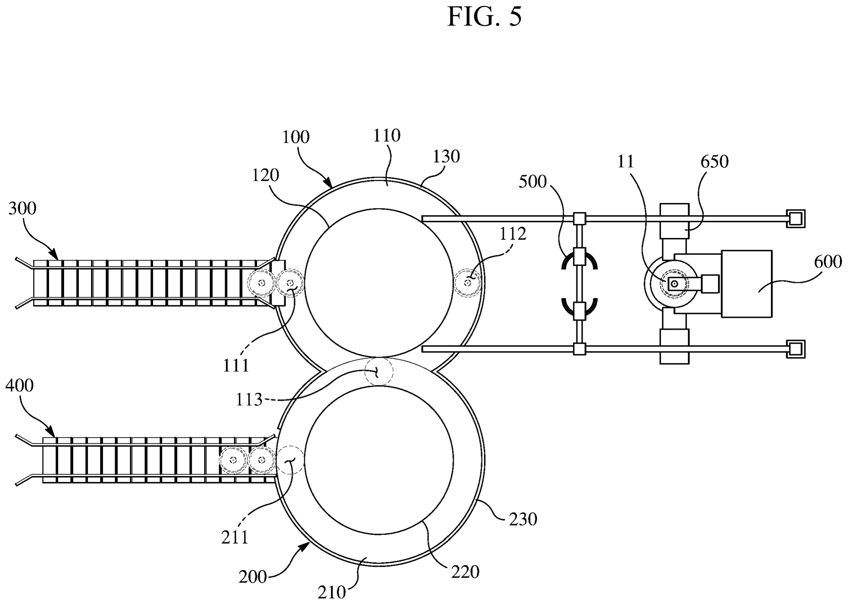

As illustrated in FIG. 5, the analysis target battery 11 may be accommodated in the gas extraction unit 600, and in the gas extraction unit 600, as illustrated in FIG. 9, a clump 650 may further fix the state in which a lower jig 620 and an upper jig 610 are coupled. A punching part 630 may form a perforation hole in the analysis target battery 11, and the analysis target gas may be extracted from the analysis target battery 11.

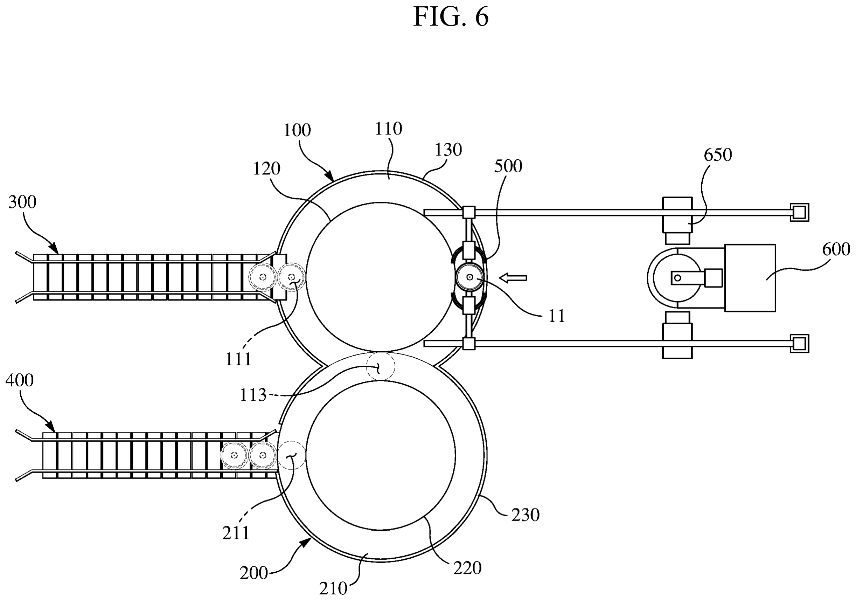

As illustrated in FIG. 6, the analysis target battery 11 from which the analysis target gas has been extracted may be moved back to the second position 112 by the gripper unit 500.

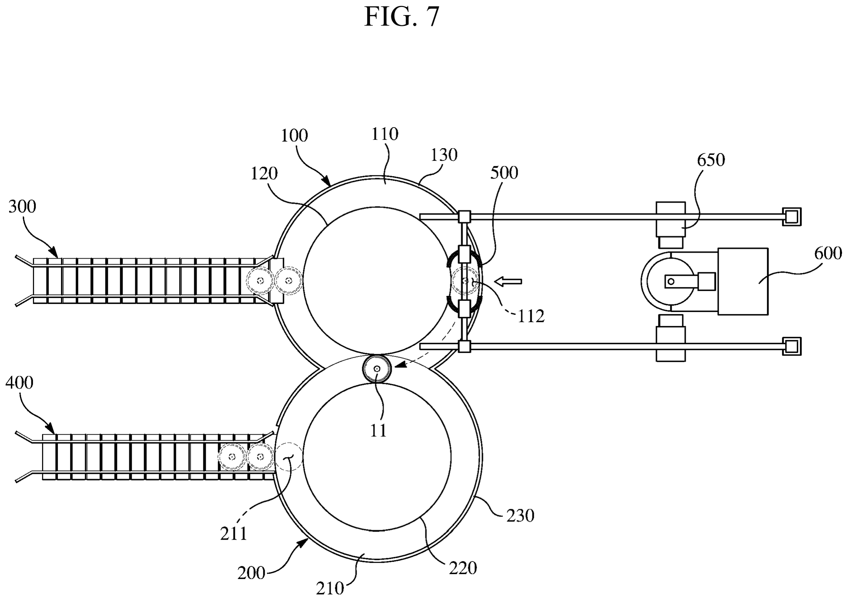

As illustrated in FIG. 7, the analysis target battery 11 may be moved from the second position 112 to the fourth position 113 by the first rotating part 110.

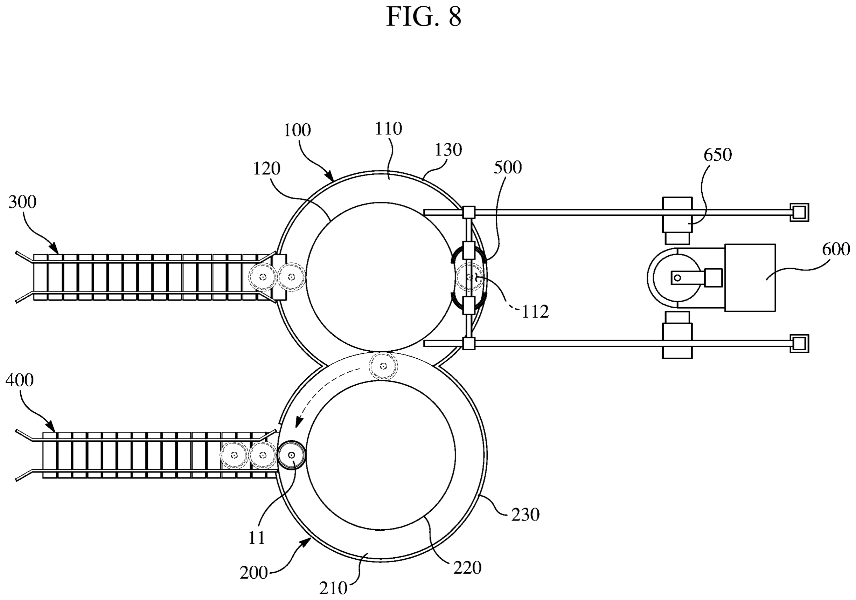

As illustrated in FIG. 8, the analysis target battery 11 may be moved from the fourth position 113 to the third position 211 by the second rotating part 210 and unloaded by the unloading unit 400.

As illustrated in FIG. 9, the gas extraction unit 600 may include:
the lower jig 620 into which the lower end part of the analysis target battery 11 is inserted;
the upper jig 610 configured to cover the upper end part of the analysis target battery 11 and couple with the upper end part of the lower jig 620;
the punching part 630 mounted on the upper jig 610 and configured to form the perforation hole on the upper surface of the analysis target battery 11;
a jig moving part 640 configured to move the upper jig 610 in the vertical direction; and
the clump 650 configured to maintain the upper jig 610 and the lower jig 620 in a coupled state.

The upper jig 610 and the lower jig 620 may be formed in a cylindrical shape extending in the vertical direction.

A cylindrical shaped groove may be formed on the bottom surface of the upper jig 610 and the upper surface of the lower jig 620, and the groove may be formed as a battery accommodation space 601.

At the lower end part of the upper jig 610, a first protrusion part 611 protruding in the radial direction along the outer circumferential surface may be formed, and at the upper end part of the lower jig 620, a second protrusion part 621 protruding in the radial direction along the outer circumferential surface may be formed.

On the bottom surface of the first protrusion part 611 or the upper surface of the second protrusion part 621, an O-ring insertion groove may be formed. The bottom surface of the first protrusion part 611 and the upper surface of the second protrusion part 621 may be in close contact with each other, so that the battery accommodation space 601 may be formed as a sealed space.

The clump 650 may be configured to hold the first protrusion part 611 and the second protrusion part 621 so as to be in close contact with each other in a state that the bottom surface of the first protrusion part 611 and the upper surface of the second protrusion 621 are in close contact with each other.

The clumps 650 may be provided in a pair, and a pair of the clumps 650 may hold opposite ends of each other.

The jig moving part 640 may be a pneumatic cylinder.

The gas extraction unit 600 may further include a cleaning unit 800 configured to clean the inner side of the upper jig 610 and a punching needle of the punching part 630. The cleaning unit 800 may be for removing the electrolyte that is stuck on the inner side of the upper jig 610 and the punching needle of the punching part 630. The punching needle may be configured to perforate the case of the analysis target battery 11.

As illustrated in FIGS. 10 to 12, the cleaning unit 800 may include:
a nozzle part 810 configured to spray air;
a friction block 820 configured to clean through friction;
a first frame 830 having one end part coupled to the nozzle part 810 and the other end part coupled to the friction block 820;
a first rail 840 configured to guide the first frame 830 to move in the longitudinal direction of the first frame 830;
a second frame 850 extending in a direction different from the longitudinal direction of the first frame 830; and
a second rail 860 configured to guide the second frame 850 to move in the longitudinal direction of the second frame 850.

The first rail 840 may be fixed to the second frame 850.

The first frame may be approached or spaced apart from the upper jig 610 by movement of the second frame 850.

The first frame 830 and the second frame 850 may be provided with a driving module such as a motor.

The first frame 830 and the first rail 840 may select one of the nozzle part 810 that sprays air and the friction block 820 that cleans through friction depending on the degree of contamination.

The second frame 850 and the second rail 860 may be for approaching and retracting to the upper jig 610.

The gas collecting apparatus of the present disclosure may further include a gas diffusion unit 700 having a gas diffusion space 721 configured to receive the analysis target gas from the gas extraction unit 600 and diffuse the analysis target gas.

As illustrated in FIGS. 13 to 15, the gas diffusion unit 700 may include:
a base plate part 710 formed as a plane perpendicular to the vertical direction;
a cylindrical side wall part 720 which has a lower end part fixed to the base plate part 710 and is capable of being stretched in the vertical direction;
a vertical moving part 730 to which the upper end part of the cylindrical side wall part 720 is fixedly coupled and configured to move in the vertical direction;
a guide support part 740 configured to guide the movement of the vertical moving part 730 in the vertical direction; and
a vertical driving part 750 configured provide the driving force for moving the vertical moving part 730 in the vertical direction.

A space surrounded by the base plate part 710, the cylindrical side wall part 720, and the vertical moving part 730 may form the gas diffusion space 721.

The base plate part 710 may be a plate having a plane shape perpendicular to the vertical direction and may be a plate made of a rigid material. For example, the material of the base plate part 710 may be SUS.

A gas port 711 is formed in the base plate part 710, and the analysis target gas may be injected into or discharged from the gas diffusion space 721 through the gas port 711. The center of the gas port 711 may be located at the center of the circular area facing the cylindrical side wall part 720 among the areas of the base plate part 710. The first gas flow path 12 and the second gas flow path 13 may be connected to the gas port 711. The flow path connected to the gas port 711 may include a hose, a tube, a pipe, etc.

The cylindrical side wall part 720 may be provided in a cylindrical shape with the vertical direction as the central axis. The cylindrical side wall part 720 may be extended or contracted in the vertical direction. The inner diameter value of the cylindrical side wall part 720 may be fixed when extended or contracted in the vertical direction. For example, the cylindrical side wall part 720 may be a bellows structure.

The vertical moving part 730 may include a body member 731 with a cylindrical shape extending in the vertical direction, and an upper plate member 732 formed as a plane perpendicular to the vertical direction and having a bottom surface to which the upper end part of the body member 731 is fixedly coupled.

The upper end part of the cylindrical side wall part 720 may be fixedly coupled to the bottom surface of the upper plate member 732, and the body member 731 may be located inside the cylindrical side wall part 720. A space surrounded by the lower end part of the body member 731, the upper surface of the base plate part 710, and the inner circumferential surface of the cylindrical side wall part 720 may form the gas diffusion space 721. Here, an ideal structure may be a structure in which the inner diameter of the cylindrical side wall part 720 and the outer diameter of the body member 731 are the same, and the coefficient of friction between the outer circumferential surface of the body member 731 and the inner circumferential surface of the cylindrical side wall part 720 may be zero (0).

When the vertical moving part 730 is lowered to the lowest point, the lower end part of the body member 731 may contact the upper surface of the base plate part 710, and all inner circumferential surfaces of the cylindrical side wall part 720 may face the outer circumferential surface of the body member 731. In other words, in the ideal structure, the volume of the gas diffusion space 721 may be zero (0) in the maximum lowered state of the vertical moving part 730 as shown in FIG. 15.

The upper plate member 732 may be formed in a disk shape, the guide support part 740 may be formed in a cylindrical shape extending in the vertical direction, the inner diameter of the guide support part 740 may be the same as the diameter of the upper plate member 732, and the upper plate member 732 may be guided by sliding on the inner circumferential surface of the guide support part 740. For example, in the ideal state, the coefficient of friction between the side surface of the upper plate member 732 and the inner circumferential surface of the guide support part 740 may be 0. The upper plate member 732 may be formed to have a predetermined thickness to prevent the position from being twisted when moving in the vertical direction.

The lower end part of the guide support part 740 may be fixed to the upper surface of the base plate part 710, and a guide hole 741 extending in the vertical direction may be formed on the side surface of the guide support part 740.

The vertical driving part 750 may include a power transmission member 751 having one end part inserted into the guide support part 740 through the guide hole 741 and the other end part located outside the guide support part 740, a vertical moving shaft 752 extending in the vertical direction and coupled to the other end of the power transmission member 751, and a driving actuator 753 that is supported on the base plate part and configured to move the vertical moving shaft 752 in the vertical direction.

The power transmission member 751 may be provided as a support made of a rigid material and in a rod shape extending in a direction perpendicular to the vertical direction.

The power transmission member 751 may be coupled to the upper surface of the upper plate member 732. More specifically, the bottom surface of the one end part side of the power transmission member 751 inserted into the guide support part 740 through the guide hole 741 may be attached to the upper surface of the upper plate member 732.

The gas diffusion unit having the above configuration may vary the volume of the gas diffusion space when sampling the analysis target gas diffused in the gas diffusion space by the sampling unit, thereby enabling sampling of the analysis target gas at a concentration optimized for analysis in the gas analysis unit.

Although embodiments according to the present disclosure have been described above, they are only illustrative and those skilled in the art will understand that various modifications and embodiments of equivalent range are possible therefrom. Therefore, the true technical protection scope of the present disclosure should be defined by the appended claims.

### <Explanation of Symbols>

11...Analysis target battery 12...First gas flow path 13...Second gas flow path 100...First moving unit 110...First rotating part 111...First position 112...Second position 113...Fourth position 120...First driving part 130...First guide part 200...Second moving unit 210...Second rotating part 211...Third position 220...Second driving part 230...Second guide part 300...Loading unit 310...Loading conveyor belt 320...Loading guide bar 400...Unloading unit 410...Unloading conveyor belt 420...Unloading guide bar 500...Gripper unit 600...Gas extraction unit 601...Battery accommodation space 610...Upper jig 611...First protrusion part 620...Lower jig 621...Second protrusion part 630...Punching part 640...Jig moving part 650...Clump 700...Gas diffusion unit 710...Base plate part 711...Gas port 720...Cylindrical side wall part 721...Gas diffusion space 730... Vertical moving part 731...Body member 732...Upper plate member 740...Guide support part 741...Guide hole 750... Vertical driving part 751...Power transmission member 752...Vertical moving shaft 753...Driving actuator 800...Cleaning unit 810...Nozzle part 820...Friction block 830...First frame 840...First rail 850...Second frame 860...Second rail

## Claims

1. A gas collecting apparatus, comprising:
a loading unit configured to sequentially supply a plurality of batteries, one by one, to a first position on a first moving unit, wherein the first moving unit is configured to move a battery of the plurality of batteries located at the first position to a second position on the first moving unit;
a gripper unit configured to position the battery located at the second position in a gas extraction unit or move the battery positioned in the gas extraction unit to the second position, wherein the gas extraction unit is configured to extract gas from the battery housed therein;
a second moving unit configured to receive the battery located at the second position from the first moving unit and move the battery to a third position, wherein the third position is located on the second moving unit; and
an unloading unit configured to receive the battery located at the third position from the second moving unit.

2. The gas collecting apparatus of claim 1, wherein the first moving unit comprises:
a first rotating part configured to rotate around a first center as a central axis; and
a first driving part configured to rotate the first rotating part.

3. The gas collecting apparatus of claim 2, wherein the first rotating part has a disk shape, and
the first moving unit further comprises a first guide part configured to restrict the battery from deviating from a circumferential direction of the first rotating part.

4. The gas collecting apparatus of claim 3, wherein the loading unit comprises:
a loading conveyor belt configured to sequentially transport the plurality of batteries to an upper surface of the first rotating part; and
a loading driver configured to drive the loading conveyor belt,
wherein an upper surface of the loading conveyor belt is higher than an upper end part of the first guide part.

5. The gas collecting apparatus of claim 4, wherein the loading unit further comprises a pair of loading guide bars configured to guide the plurality of batteries so as to be aligned along a longitudinal direction of the loading conveyor belt and restrict the plurality of batteries from leaving the loading conveyor belt, and
wherein the pair of loading guide bars are parallel to each other, spaced apart from each other by a distance greater than or equal to a diameter of each battery of the plurality of batteries, and extend in the longitudinal direction of the loading conveyor belt.

6. The gas collecting apparatus of claim 4, wherein the loading conveyor belt is configured to transport the plurality of batteries in a first direction, and
the second moving unit comprises:
a second rotating part configured to rotate around a second center as a central axis that is spaced apart from the first center by a predetermined distance in a second direction, the second direction being perpendicular to the first direction and parallel to the upper surface of the first rotating part; and
a second driving part configured to rotate the second rotating part.

7. The gas collecting apparatus of claim 6, wherein the second rotating part has a disk shape, and
the predetermined distance is smaller than a sum of a radius of the first rotating part and a radius of the second rotating part.

8. The gas collecting apparatus of claim 7, wherein the predetermined distance is a value obtained by subtracting a diameter of the battery from the sum of the radius of the first rotating part and the radius of the second rotating part,
the second moving unit further comprises a second guide part configured to restrict the battery from deviating from a circumferential direction of the second rotating part, and
the first rotating part and the second rotating part overlap at a fourth position, the fourth position being a position where the first guide part and the second guide part are open to each other.

9. The gas collecting apparatus of claim 8, wherein the unloading unit comprises:
an unloading conveyor belt configured to sequentially receive the plurality of batteries from the second rotating part; and
an unloading driver configured to provide driving force to the unloading conveyor belt,
wherein an upper surface of the unloading conveyor belt is lower than an upper surface of the second rotating part, and
wherein the second guide part is open in an area of the second rotating part that is in contact with one end part of the unloading conveyor belt.

10. The gas collecting apparatus of claim 9, wherein the first position is a position where the battery loaded by the loading conveyor belt onto the first rotating part is first placed,
the first rotating part is configured to rotate in a direction such that the battery located at the first position moves away from the fourth position,
the second rotating part is configured to rotate in an opposite direction to the first rotating part, and
at the fourth position, the battery is configured to move from the first rotating part to the second rotating part by the rotation of the second rotating part.

11. The gas collecting apparatus of claim 10, wherein the second position is a position on the first rotating part located between the first position and the fourth position, and
the third position is a position of an edge of the second rotating part where the second rotating part is in contact with the one end part of the unloading conveyor belt.

12. The gas collecting apparatus of claim 1, wherein the gas extraction unit comprises:
a lower jig into which a lower end part of the battery is configured to be inserted;
an upper jig configured to cover an upper end part of the battery and couple with an upper end part of the lower jig;
a punching part mounted on the upper jig and configured to form a perforation hole on an upper surface of the battery;
a jig moving part configured to move the upper jig in a vertical direction; and
a clump configured to maintain the upper jig and the lower jig in a coupled state.

13. The gas collecting apparatus of claim 12, wherein each of the upper jig and the lower jig has a cylindrical shape extending in the vertical direction,
a first protrusion part protruding in a radial direction along an outer circumferential surface is formed at a lower end part of the upper jig,
a second protrusion part protruding in a radial direction along an outer circumferential surface is formed at the upper end part of the lower jig, and
the clump is configured to hold the first protrusion part and the second protrusion part so as to be in close contact with each other in a state that a bottom surface of the first protrusion part and an upper surface of the second protrusion part are in close contact with each other.

14. The gas collecting apparatus of claim 12, further comprising:
a cleaning unit configured to clean an inner side of the upper jig and a punching needle of the punching part,
wherein the cleaning unit comprises:
a nozzle part configured to spray air;
a friction block configured to clean through friction;
a first frame having a first end part coupled to the nozzle part and a second end part coupled to the friction block;
a first rail configured to guide the first frame to move in a longitudinal direction of the first frame;
a second frame extending in a direction different from the longitudinal direction of the first frame; and
a second rail configured to guide the second frame to move in a longitudinal direction of the second frame, and
wherein the first rail is fixed to the second frame, and
the first frame is configured to be moved toward or away from the upper jig by movement of the second frame.

15. The gas collecting apparatus of claim 1, further comprising:
a gas diffusion unit having a gas diffusion space configured to receive the extracted gas from the gas extraction unit and diffuse the gas,
wherein the gas diffusion unit comprises:
a base plate part having a plane shape perpendicular to a vertical direction;
a cylindrical side wall part having a lower end part fixed to the base plate part, the cylindrical side wall part configured to be stretched in the vertical direction;
a vertical moving part configured to move in the vertical direction, wherein an upper end part of the cylindrical side wall part is fixedly coupled to the vertical moving part;
a guide support part configured to guide a movement of the vertical moving part in the vertical direction; and
a vertical driving part configured to move the vertical moving part in the vertical direction, and
wherein the gas diffusion space is defined by the base plate part, the cylindrical side wall part, and the vertical moving part.
